Europäisches Patentamt

**European Patent Office**

**Office européen des brevets**

(19)

(11) Publication number: **0 095 786**
**A1**

# EUROPEAN PATENT APPLICATION

(12)

(21) Application number: **83200427.9**

(22) Date of filing: **26.03.83**

(51) Int. Cl.³: **C 12 M 1/26**

(30) Priority: **24.05.82 US 381361**

(43) Date of publication of application: **07.12.83**
**Bulletin 83/49**

(84) Designated Contracting States: **BE DE FR GB LU NL**

(71) Applicant: **Maddak, Inc., Industrial Road, Pequannock**
**New Jersey (US)**

(72) Inventor: **Broeils, John, 188 Cedar Hill Avenue, Wycoff**
**New Jersey, 07481 (US)**
Inventor: **Milden, Morton, 19 Colonial Woods Drive, West**
**Orange New Jersey, 07052 (US)**
Inventor: **Petersen, Howard E., Jr., 19 Kastan Avenue,**
**Staten Island New York, 10308 (US)**

(74) Representative: **Noz, Franciscus Xaverius, Ir. et al,**
**Boschdijk 155, NL-5612 HB Eindhoven (NL)**

(54) **Electrically heated inoculating loop system.**

(57) An electrically heated innoculating loop system features a power supply (2) including means (46) for controlling the current to heat an inoculating loop. A holder (16) for the loop includes a member (14) cooperating with means (30, 32) arranged with the loop for interchangeably retaining loops of various sizes and shapes, and said member and means further cooperating to draw heat away from the holder when the loop is heated.

EP 0 095 786 A1

-1-

ELECTRICALLY HEATED INNOCULATING LOOP SYSTEM

Innoculating loop systems include a wire which has been looped at one end and sterilized so that a user can transport bacteriological cultures or specimens from one container to another without contaminating the culture or specimen. The looped wire is electrically heated to achieve the required sterilization quickly and uniformly. Apparatus of the type for accomplishing this purpose is described in U.S. Patent 4,242,462 issued on December 30, 1980 to Richard E. Thomas.

In order for electrically heated innoculating loop systems of the type described to have maximum utility and efficiency, they must accommodate loop wires which are formed in a variety of shapes and sizes and which may be used interchangeably. Additionally, it is desirable to maximize user safety by controlling the electrical current which heats the looped wire and by keeping the ensuing heat away from the users hand.

Accordingly, it is an object of the present invention to provide an electrically heated innoculating loop system including a holder for interchangeably retaining looped wires of various sizes and shapes and means for drawing the heat away from the holder when the looped wire is heated, and further including a power supply including current controlling means for heating the looped wire.

This invention contemplates an electrically heated innoculating loop system including an electrical power supply, and an innoculating loop holder including means for retaining the innoculating loop and for connecting the loop to the power supply. Said means includes a socket having socket terminals and supported by the holder for interchangeably receiving innoculating loops of various sizes and shapes. The socket and socket terminals cooperate with terminals on the loop to draw heat away from the holder when the innoculating loop is heated. The power supply includes current controlling means whereby the electrical current to which the user is exposed is limited to a safe level.

Figure 1 is a diagrammatic representation showing an electrically heated innoculating loop system according to the invention.

Figure 2 is a diagrammatic representation showing a holder for an electrically heated innoculating loop and a socket member arranged with the holder for removably retaining the loop and for connecting the loop to a power supply, said holder and socket member being generally shown in Figure 1.

Figure 3 is a sectional view of the innoculating loop holder shown in Figures 1 and 2.

Figure 4 is a sectional view taken along the line 4-4 in Figure 3.

Figure 5 is a diagrammatic representation of a form of innoculating loop shown generally in Figures 1 and 2.

Figure 6 is an end view of a socket member for the innoculating loop also shown generally in Figure 1 and 2.

Figure 7 is an electrical schematic of the electrically heated innoculating loop system shown diagrammatically in Figure 1.

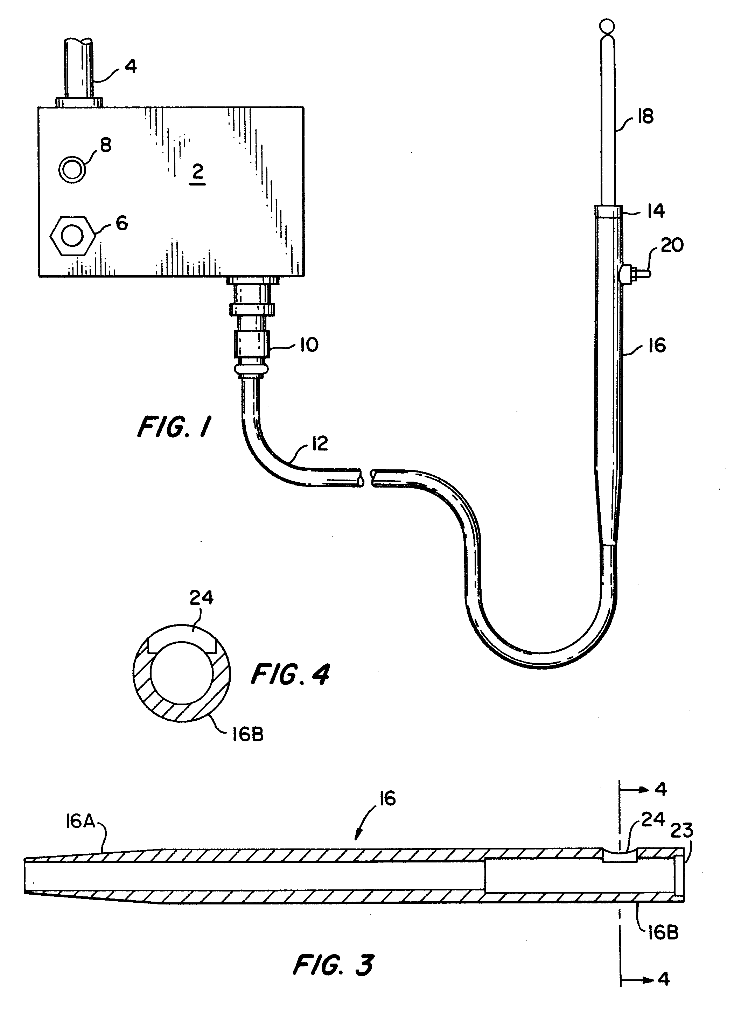

With reference first to Figure 1, a power supply is designated generally by the numeral 2 and is connected through a cable 4 to a suitable voltage source (not shown). A switch 6 is operator-operable for turning the power supply on and off and an indicating lamp 8 indicates as by lighting when the power supply is on, and indicates as by lightning when the power supply is off.

Power supply 2 is connected through a suitable electrical connector 10 and a cable 12 to a socket member 14 carried by an innoculating loop holder 16. As will be hereinafter described, socket member 14 removably retains an innoculating loop 18.

Innoculating loop holder 16 carries a switch 20, connected to power supply 2 via cable 12, which may be operator-operated for allowing electrical current to flow from power supply 2 through cable 12 and socket member 14 to innoculating loop 18 to heat the loop, or to prevent said current flow, as will be hereinafter become evident.

With reference to Figure 2, innoculating loop holder 16 surrounds the end of cable 12 opposite connector 10, and which cable end exposes a pair of electric conductors 22 and 24 and another pair of electrical conductors 26 and 28.

The structural arrangement is such that switch 20, which is supported by innoculating loop holder 16 as will hereinafter be further described, is connected via a pair of terminals 20C to conductors 26 and 28, while socket member 14 has terminals 14A and 14B which are connected to conductors 22 and 24, respectively, whereby switch 20 and socket member 14 are connected to power supply 2.

With reference to Figures 3 and 4, innoculating loop holder 16 is a generally cylindrical, substantially hollow elongated member having a tapered end 16A which receives the end of cable 12 opposite the end of the cable connected to connector 10 (Figures 1, 2) and an opposite end 16B having a longitudinal recess 23 adapted for receiving socket member 14 (Figure 2).

Holder 16 has a circumferential opening 24, as best shown in Figure 4, for receiving switch 20. In this connection, reference is again made to Figure 2 wherein switch 20 has a push button 20A, a housing 20B and the aforenoted pair of terminal members 20C. Housing member 20B is disposed in opening 24 and is secured therein as by cementing or the like, whereby terminal members 20C extend internal to holder 16 and push button 20A extends external thereto.

Switch 20 will be recognized as being of a commercially available type which is widely used in the electrical arts. Holder 14 may be fabricated from a suitable rigid material which may be aluminum or some other such suitable material.

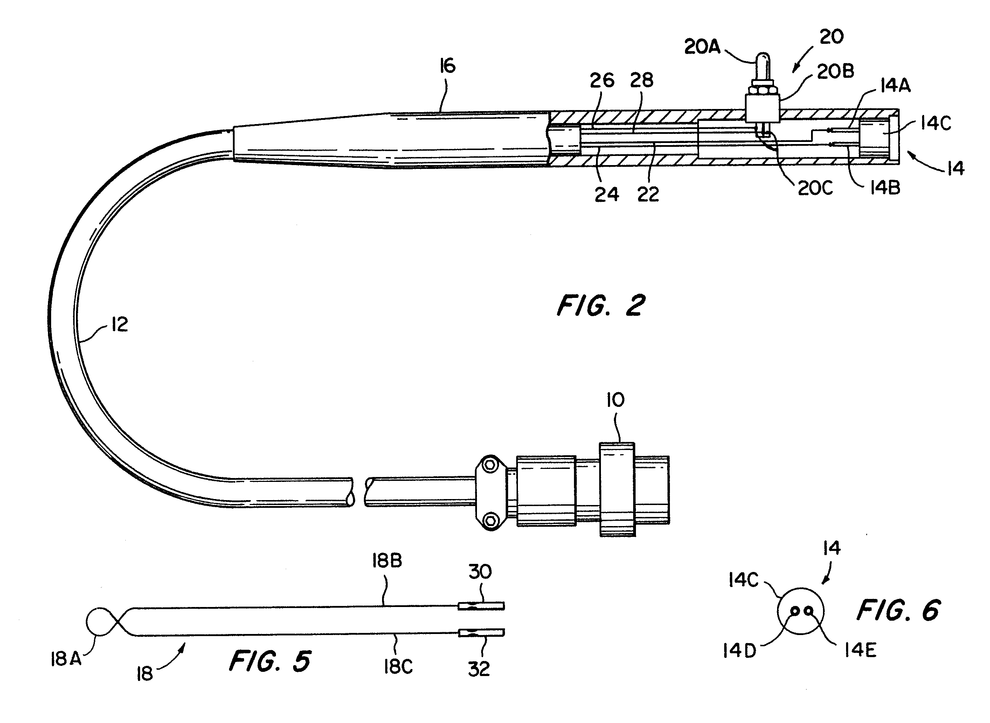

With reference to Figure 5, innoculating loop 18, in one form, may be of a suitable wire such as Nichrome No. 25, approximately .02 inches in diameter, two and one-half inches long and having a resistance of approximately two ohms per foot. The wire is formed so as to have a loop 18A on one end thereof which serves to transport the aforementioned bacteriological specimens or cultures from one container to another, and to have terminal ends 18B and 18C, respectively.Terminals 30 and 32 which are secured to ends 18B and 18C, respectively, are commercially available terminals such as manufactured by Auget, Inc. under the manufactures part number LST-1AG9-19.

It will now be understood that only one shape and size of innoculating loop 18 is shown for illustrative purposes. Other sizes and shapes are within the contemplation of the invention as well, as will now be understood.

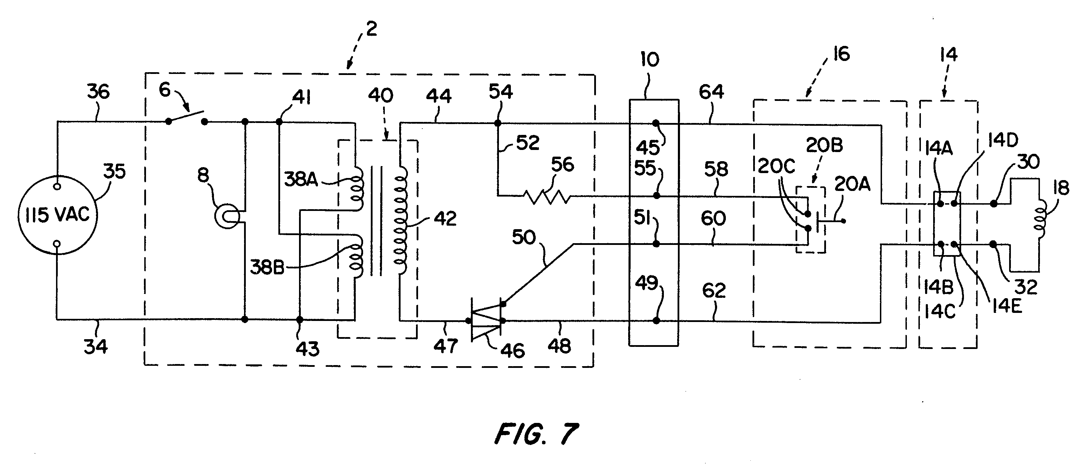

With reference to Figure 6, socket member 14 has an insulating base 14C which supports conductive (metallic) terminals 14A and 14B (Figure 2) on one side thereof. The other size of base 14C includes sockets 14D and 14E connected to terminals 14A and 14B, respectively. Base 14C press fits into recess 23 of holder 14 (Figure 3). Sockets 14D and 14E receive terminals 30 and 32 on wire loop ends 18B and 18C in "push-fit" or removeable relationship. A socket member of the type described suitable for the purposes of the invention is manufactured by Auget, Inc. under the manufactures part number 8060-1G25.

With reference to Figure 7, cable 4 includes a conductor 34 and a conductor 36 connected to a suitable voltage source 35 as a 115 volt a.c. source. Conductor 36 is connected through switch 6 to one leg of a primary winding 38A of a transformer 40, and conductor 34 is connected to one leg of a primary winding 38B. Another leg of primary winding 38B is connected to conductor 36 at a circuit point 41 intermediate the primary winding and switch 6, and another leg of primary winding 38A is connected to conductor 34 at a circuit point 43 intermediate the primary winding and the voltage source. Lamp 8 is connected across conductors 34 and 36 intermediate switch 6 and circuit point 41, and intermediate the voltage source and circuit point 43.

Transformer 40, which may be a conventional iron core transformer, has a secondary winding 42, inductively coupled to primary windings 38A, 38B. One end of secondary winding 42 is connected through a conductor 44 to connector 10 at a circuit point 45 and the other end of secondary winding 42 is connected through a conductor 47 to a current control device shown as a convential triac 46. Triac 46 is connected through conductors 48 and 50 to connector 10 at circuit points 49 and 51, respectively.

A conductor 52 is connected to conductor 44 at a circuit point 54 intermediate secondary winding 42 and circuit point 45, and is connected to connector 10 at a circuit point 55 through a balance resistor 56.

It will now be understood that switch 6, lamp 8, transformer 40, triac 46 and resistor 56, and the associated conductors, are included in power supply 2 shown in Figure 1.

A conductor 58 is connected to circuit point 55 and a conductor 60 is connected to circuit point 51. Another conductor 62 is connected to circuit point 49, while yet another conductor 64 is connected to circuit point 45.

Conductors 58 and 60 are connected to terminal member 20C of switch 20, which is mounted to holder 16 as heretofore described. Conductor 62 is connected to terminal 14B of socket member 14 and conductor 64 is connected to terminal 14A of the socket member. Innoculating loop 18, which provides a load for the described circuitry is connected at terminal 30 to socket 14D which, in turn, is connected to terminal 14A, and is connected at terminal 32 to socket 14E which, in turn, is connected to terminal 14B, as shown in Figure 7.

With the above description in mind, the operation of the invention will now be described. Switch 6 is normally open and lamp 8 does not light, indicating that no power is flowing to the system. When switch 6 is manually closed, power from 115 volt a.c. source 35 flows to the system and lamp 8 lights, being indicative thereof. Transformer 40 transforms the 115 volts at its primary windings 38A, 38B to, for example, twelve volts at its secondary winding 42.

Switch 20, mounted to holder 14, is normally open and when closed permits current to flow to socket 14 and therefrom to coil 18 to heat the coil and to thereby sterilize the coil for accomplishing the purposes of the invention. The arrangement of triac 46 is such that the closing of switch 20 triggers the triac, which thereupon controls the current at the switch to, for example, nine amperes.

It will now be seen that the aforementioned purposes of the invention have been accomplished. Means (triac 46) are provided for controlling the current which heats innoculating loop 18 so as to enhance the safety of the system. Structure is provided for easily removing and interchanging innoculating loops as is a distinct advantage. Terminals 30 and 32 in cooperation with socket 14, which is constructed of metal and a suitable insulating material is effective for absorbing heat away from heated loop 18 to keep the heat away from holder 16 and hence the operators hand, which also enhances the safety of the system.

The figures used in the claims are only meant to explain more clearly the intention of the invention and are not supposed to be any restriction concerning the interpretation of the invention.

-1-

CLAIMS:

1.      An electrically heated innoculating loop system, comprising:

a wire member of a material capable of being electrically heated and formed into an innoculating loop which terminates in a pair of elongated legs;

a pair of electrical terminals, each of which is coupled to a corresponding leg;

a handle for the innoculating loop;

socket means supported by the handle and having a pair of electrical sockets;

each of the electrical terminals removably engaging a corresponding electrical socket for removably coupling the innoculating loop to the socket means.

a power supply connected to the socket means for supplying electrical current to the socket means for heating the loop when the loop is coupled to the socket means; and

the power supply including current control means for controlling the current to the socket means.

2.          A system as described by Claim 1 wherein:

the handle is a hollow, elongated member having opposite open ends;

the socket means is supported in one of the open ends and has a pair of socket terminals, each of which is coupled to a corresponding socket and extends within the handle;

the electrical power supply includes electrical conductor means extending through the opposite handle end, and connected to one of  the pair of  socket terminals extending within the handle.

3.          A system as  described by Claim 2, including:

normally open switching means supported in a circumferential opening in the handle and exterior thereto, and having terminal means extending with the handle;

the electrical power supply includes other electrical conductor means extending through the opposite handle end and connected  to the terminal means;

the current control means of the power supply connected to the terminal means  and to the socket means; and

the normally open switching means operator-operable for being closed, whereby the current control means is triggered for controlling the electrical current to the switching means and to the socket means.

4.          A system  as described by Claim 1,  wherein:

the socket means supported by the handle cooperating with the pair of electrical terminals for drawing heat away from the handle when the loop is electrically heated.

5.          A system as described by Claim 3, wherein:

the electrical power supply includes a transformer having a primary winding connected to a voltage cource  and a secondary winding inductively coupled to the primary winding;

the electrical conductor means extending through the opposite handle end and connected to one of the pair of socket terminals in connected to one end of the secondary winding;

the other electrical conductor means extending through the opposite handle end and connected to the terminal means  being connected to the first mentioned electrical conductor means between the one end of the secondary winding and the terminal means; and

the current control means being connected to the other end of the secondary winding.

6. A system as described by Claim 2, wherein:

the current control means includes first conductor means connected to the terminal means and second conductor means connected to the other of the pair of socket terminals extending within the handle.

7. An electrically heated innoculating loop system comprising:

a wire member of a material capable of being electrically heated and formed into an innoculating loop which terminates in a pair of elongated legs;

a pair of electrical terminals, each of which is coupled to a corresponding leg;

a substantially hollow elongated handle having opposite open ends;

socket means supported in one of the open ends and including a pair of sockets and a pair of socket terminals each of which is coupled to a corresponding socket and extends within the handle;

a normally open switch supported on the handle exterior thereto and having terminal means extending within the handle;

a power supply connected within the handle to one of the pair of socket terminals and to the terminal means;

the power supply including current control means;

the current control means connected within the handle to the other of the pair of sockets and to the terminal means, and effective for controlling the current to the switch and to the socket means upon closure of the switch; and

the electrical terminals coupled to the legs of the loop removably engaging corresponding sockets of the pair of sockets, whereby the loop is heated by the controlled current upon closure of the switch.

FIG. 1

FIG. 4

FIG. 3

*FIG. 2*

*FIG. 6*

*FIG. 5*

FIG. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | PATENTS ABSTRACTS OF JAPAN, vol. 5, no. 8 (C-39), 20th January 1981, page 680 C 39, Tokyo, JP. & JP - A - 55 135 585 (NIPPON KIHATSUYU K.K.) 22-10-1980 * Abstract, figure * | 1,5,7 | C 12 M   1/26 |
| | --- | | |
| Y | PATENTS ABSTRACTS OF JAPAN, vol. 5, no. 8 (C-39), 20th January 1981, page 680 C 39, Tokyo, JP. | 2-3 | |
| | --- | | |
| D | US-A-4 242 462 (R.E. THOMAS) * Figures; claims; column 2, line 41 - column 3, line 9; column 3, line 51 - column 4, line 68 * | 2,3,5 | |
| Y | | 7 | |
| | | | **TECHNICAL FIELDS SEARCHED (Int. Cl. 3)** |
| | --- | | |
| A | US-A-4 074 110 (P.E. SLAUGHTER) * Figure 3; column 4, lines 29-47 * | 4 | C 12 M |
| | --- | | |
| A | US-A-3 461 874 (M. MARTINEZ) | | |
| | ----- | | |

The present search report has been drawn up for all claims

| Place of search | Date of completion of the search | Examiner |
|---|---|---|
| THE HAGUE | 07-09-1983 | COUCKE A.O.M. |